(19) Europäisches Patentamt
European Patent Office
Office européen des brevets

(11) **EP 2 247 744 B1**

(12) **FASCICULE DE BREVET EUROPEEN**

(45) Date de publication et mention de la délivrance du brevet:
**08.06.2016 Bulletin 2016/23**

(21) Numéro de dépôt: **09710311.3**

(22) Date de dépôt: **12.02.2009**

(51) Int Cl.:
*C12P 21/06* (2006.01)     *A23J 3/34* (2006.01)
*A61K 35/60* (2006.01)     *A61K 38/01* (2006.01)
*C07K 14/595* (2006.01)     *A23J 3/04* (2006.01)
*A23L 33/175* (2016.01)

(86) Numéro de dépôt international:
**PCT/EP2009/051638**

(87) Numéro de publication internationale:
**WO 2009/101134 (20.08.2009 Gazette 2009/34)**

(54) **Hydrolysat de protéines de poissons présentant une activité satiétogène, compositions nutraceutiques et pharmacologiques comprenant un tel hydrolysat et procédé d'obtention**

Fischproteinhydrolysat mit Sättigungswirkung, nutrazeutische und pharmakologische Zusammensetzungen mit einem derartigen Hydrolysat, sowie Verfahren zu seiner Gewinnung

Fish protein hydrolysate having a satietogenic activity, nutraceutical and pharmacological compositions comprising such a hydrolysate and method for obtaining same

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **12.02.2008 FR 0800751**

(43) Date de publication de la demande:
**10.11.2010 Bulletin 2010/45**

(73) Titulaires:
• **COMPANIE DES PECHES SAINT MALO SANTE**
**35400 Saint-Malo (FR)**
• **MUSEUM NATIONAL D'HISTOIRE NATURELLE**
**75005 Paris (FR)**
• **CENTRE NATIONAL DE LA RECHERCHE SCIENTIFIQUE**
**75794 Paris Cedex 16 (FR)**
• **UNIVERSITE DE LILLE 1 - SCIENCES ET TECHNOLOGIES**
**59655 Villeneuve d'Ascq Cedex (FR)**

(72) Inventeurs:
• **DRIEU LA ROCHELLE, Hubert**
**F-35400 Saint-Malo (FR)**
• **COUROIS, Elisa**
**F-35400 Saint-Malo (FR)**
• **CUDENNEC, Benoît**
**F-29000 Quimper (FR)**

• **FOUCHEREAU-PERON, Martine**
**F-29910 Tregunc (FR)**
• **RAVALLEC-PLE, Rozenn**
**F-59120 Loos (FR)**

(74) Mandataire: **Le Guen-Maillet**
**5, place de Newquay**
**BP 70250**
**35802 Dinard Cedex (FR)**

(56) Documents cités:
WO-A-2004/016098     WO-A-2005/002605
WO-A-2006/084383     GB-A- 2 136 002

• **RAVALLEC-PLÉ ROZENN ET AL: "Secretagogue activities in cod (Gadus morhua) and shrimp (Penaeus aztecus) extracts and alcalase hydrolysates determined in AR4-2J pancreatic tumour cells." COMPARATIVE BIOCHEMISTRY AND PHYSIOLOGY. PART B, BIOCHEMISTRY & MOLECULAR BIOLOGY, vol. 134, no. 4, avril 2003 (2003-04), pages 669-679, XP002501880 ISSN: 1096-4959**
• **RAVALLEC-PLÉ ROZENN ET AL: "The presence of bioactive peptides in hydrolysates prepared from processing waste of sardine (Sardina pilchardus)" JOURNAL OF THE SCIENCE OF FOOD AND AGRICULTURE, vol. 81, no. 11, 1 septembre 2001 (2001-09-01), pages 1120-1125, XP002501882 ISSN: 0022-5142**

- GUÉRARD FABIENNE ET AL: "Fish and shellfish upgrading, traceability" ADVANCES IN BIOCHEMICAL ENGINEERING / BIOTECHNOLOGY, SPRINGER-VERLAG, GERMANY, vol. 96, 2005, pages 127-163, XP002504352 ISSN: 3-540-25659-8(H)
- LIASET BJOERN ET AL: "Enzymatic hydrolysis of by-products from the fish-filleting industry; chemical characterisation and nutritional evaluation" JOURNAL OF THE SCIENCE OF FOOD AND AGRICULTURE, vol. 80, no. 5, avril 2000 (2000-04), pages 581-589, XP002502819 ISSN: 0022-5142
- LICEAGA-GESUALDO AND LI-CHAN: "Functional properties of fish protein hydrolysate from herring (Clupea harengus)" JOURNAL OF FOOD SCIENCE, WILEY-BLACKWELL PUBLISHING, INC, US, vol. 64, no. 6, 1 janvier 1999 (1999-01-01), pages 1000-1004, XP002206030 ISSN: 0022-1147
- LIASET ET AL: "Nutritional composition of soluble and insoluble fractions obtained by enzymatic hydrolysis of fish-raw materials" PROCESS BIOCHEMISTRY, ELSEVIER, NL, vol. 43, no. 1, 22 octobre 2007 (2007-10-22), pages 42-48, XP022392540 ISSN: 1359-5113
- NISHI TAKASHI ET AL: "Dietary protein peptic hydrolysates stimulate cholecystokinin release via direct sensing by rat intestinal mucosal cells" EXPERIMENTAL BIOLOGY AND MEDICINE (MAYWOOD), vol. 226, no. 11, décembre 2001 (2001-12), pages 1031-1036, XP002501883 ISSN: 1535-3702
- L. Zhou ET AL: "Up-regulation of Cholesterol Absorption Is a Mechanism for Cholecystokinin-induced Hypercholesterolemia", Journal of Biological Chemistry, vol. 289, no. 19, 1 April 2014 (2014-04-01), pages 12989-12999, XP055179619, ISSN: 0021-9258, DOI: 10.1074/jbc.M113.534388

**Description**

Hydrolysat de protéines de poissons présentant une activité satiétogène, compositions nutraceutiques et pharmacologiques comprenant un tel hydrolysat et procédé d'obtention.

La présente invention concerne un hydrolysat de protéines de poisson contenant des molécules immunologiquement apparentées à la famille des gastrines/cholecystokinines et pouvant exercer une activité satiétogène et réguler la prise alimentaire chez l'homme ou l'animal. L'invention concerne encore un procédé d'obtention d'un tel hydrolysat de protéines de poisson ainsi qu'une composition, un produit alimentaire, un complément alimentaire ou encore un médicament comprenant un tel hydrolysat de protéines de poisson.

La surcharge pondérale est de plus en plus observée au sein de la population et devient une préoccupation constante. Un tel phénomène est le résultat d'un déséquilibre entre l'apport énergétique moyen et la somme des dépenses énergétiques. En effet, quand l'organisme reçoit plus qu'il ne dépense, il stocke une partie de l'apport énergétique sous forme de graisses dans les adipocytes constitutives du tissu adipeux. Ces cellules grossissent et entraînent alors une surcharge pondérale visible. Elles peuvent ensuite arriver à saturation et se multiplier. On parle alors d'obésité. Dans un tel cas, la surcharge pondérale est directement responsable de problèmes de santé divers tels que des problèmes cardiovasculaires, articulaires ou métaboliques.

Les facteurs responsables de la surcharge pondérale sont de deux types: les facteurs génétiques d'une part, le mode de vie et le comportement alimentaire d'autre part. C'est sur le second type de facteur que se penchent actuellement les industries agroalimentaires et nutraceutiques en s'intéressant aux facteurs biologiques qui participent aux phénomènes physiologiques responsables du comportement alimentaire, et plus particulièrement du contrôle de la satiété. Un dérèglement de ce contrôle peut être non seulement la cause d'une surcharge pondérale, mais également celle de maladies sérieuses liées aux troubles de la conduite alimentaire telles que l'obésité, le diabète de type II, les accidents cardiovasculaires, l'hypertension, l'athérosclérose, l'hypercholestérolémie.

Les cholecystokinines, dénommés CCK par la suite, sont une famille de peptides neuroendocriniens. Elles sont sécrétées au niveau de la lumière de l'intestin grêle par des cellules entéroendocrines, ainsi qu'au niveau du système nerveux central, ce qui leur confère également un rôle dans le transfert de l'information entre le tractus gastro-intestinal et le cerveau [1, 2]. Le passage des aliments dans la partie duodénale de l'intestin grêle entraîne la sécrétion des CCK. Cette sécrétion engendre de nombreux processus physiologiques tels que la mobilité intestinale, la contraction de la vésicule biliaire, l'inhibition de la vidange gastrique, la stimulation de la sécrétion pancréatique et l'induction du phénomène de satiété [4]. La libération des CCK est due, en ordre d'importance, à l'action des composés protéiques, lipidiques et glucidiques [6].

De précédents travaux ont montré le fort potentiel satiétogène exercé par certains hydrolysats protéiques chez le rat [7, 8], le porc [5] et l'homme [9].

Le document Ravallec-Plé R. et al. (2003), Comparative Biochemistry and Physiology, Part B, vol. 134, no. 4, pages 669-679 concerne des hydrolysats de protéines de cabillaud et de crevette obtenus après hydrolyse enzymatique à l'aide de l'enzyme Alcalase (enzyme dérivée de Bacillus licheniformis). Ces hydrolysats contiennent des molécules apparentées aux gastrines/CCK.

Les demandeurs ont alors découvert que des hydrolysats de protéines, ou peptidiques, obtenus à partir de l'hydrolyse enzymatique du muscle de certains poissons possédaient des propriétés stimulatrices de la sécrétion de CCK par des cellules entéroendocrines intestinales.

Le GLP-1, glucagon-like peptide 1, est une hormone gastro-intestinale sécrétée par les cellules épithéliales de l'intestin en réponse à l'ingestion de nutriments.

Le GLP-1 régule le métabolisme des nutriments et leur élimination en augmentant la synthèse et la sécrétion d'insuline lorsque la glycémie est trop élevée (glycémie post-prandiale). En parallèle, le GLP-1 freine la libération de glucagon, hormone hyperglycémiante, *via* les îlots pancréatiques.

Le GLP-1 diminue également la motricité digestive et induit la sensation de satiété.

L'invention concerne ainsi un hydrolysat de protéines de poisson qui se caractérise en en ce qu'il est obtenu par hydrolyse enzymatique d'au moins une source de protéines choisie parmi le groupe composé des espèces de poissons pélagiques *Micromesistius poutassou, Clupea harengs, Scomber scombrus, Sardina pilchardus, Trisopterus esmarki, Trachurus spp.,* de poissons démersaux *Gadus morhua, Pollachius virens, Melanogrammus aeglefinus, Coryphaenoides rupestris,* de poissons appartenant à l'ordre des siluriformes, ladite hydrolyse enzymatique étant réalisée par un mélange d'enzymes comprenant des endopeptidases dérivées de *Bacillus amyloliquefaciens* et de *Bacillus licheniformis* et en ce qu'il présente:

- la répartition du profil moléculaire suivant : de 23 à 31 % de molécules de poids moléculaire inférieur à 300 Da, de 31 à 34 % de molécules dont le poids moléculaire est compris entre 300 et 1 000 Da, de 28 à 34 % de molécules dont le poids moléculaire est compris entre 1 000 et 3 000 Da, de 6 à 8 % de molécules dont le poids moléculaire est compris entre 3 000 et 5 000 Da et de 2 à 4 % de molécules dont le poids moléculaire est compris entre 5 000 et 10 000 Da,

- une teneur en lipides inférieure à 1 %, en pourcentage de produit brut,
- une teneur en glucides inférieure à 0,1 %, en pourcentage de produit brut,
- une teneur en protéines supérieure à 80 %, en pourcentage de produit brut,
- une teneur en matière minérale comprise entre 10 et 20 %, en pourcentage de produit brut,

et en ce qu'il contient des molécules immunologiquement apparentées aux cholecystokinines, ou CCK.

L'hydrolysat de protéines selon l'invention permet un apport de molécules de CCK exogènes. Il permet, de plus, de stimuler la sécrétion de molécules de CCK et de molécules GLP1 endogènes par des cellules intestinales. L'hydrolysat permet ainsi le contrôle de la satiété comme le démontreront les exemples qui suivent.

Selon une caractéristique de l'invention, ledit hydrolysat de protéines de poisson présente la composition en acides aminés suivante : Acide glutamique 17,4 %, Acide aspartique 11,4 %, Lysine 10,2 %, Leucine 8,4 %, Arginine 6,1 %, Alanine 6,8 %, Valine 4,7 %, Isoleucine 4,2 %, Glycine 5 %, Thréonine 4,5 %, Sérine 4,4 %, Tyrosine 3,2 %, Phénylalanine 3,9 %, Méthionine 2,5 %, Proline 3,6 %, Histidine 1,9 %, Cystine 1 '%, Tryptophane 0,8 %, en pourcentage en poids par rapport au poids total d'acides aminés.

Selon un mode préféré de réalisation de l'invention, ladite source de protéines de poisson se présente sous la forme de la pulpe du filet dudit ou desdits poissons.

Selon un autre mode de réalisation de l'invention, ledit mélange d'enzymes comprend, de plus, une endopeptidase dérivée *d'Aspergillus oryzae.*

La présente invention concerne encore un procédé d'obtention d'un hydrolysat de protéines à partir d'une source de protéines de poisson possédant des propriétés stimulatrices de la sécrétion des CCK et des GLP1 au niveau de cellules intestinales et pouvant exercer un effet satiétogène et tel que défini précédemment. Le procédé selon l'invention se caractérise en ce qu'il comprend:

- le broyage d'au moins une source de protéines choisie parmi le groupe composé des espèces de poissons *Micromesistius poutassou, Clupea harengus, Scomber scombrus, Sardina pilchandus, Trisopterus esmarki, Trachurus spp.,* de poissons démersaux *Gadus morhua, Pollachius virens, Melanograrmmus aeglefinus, Coryphaenoides rupestris,* de poissons appartenant à l'ordre des siluriformes, en présence d'eau, de manière à récupérer la pulpe de poisson,
- l'hydrolyse enzymatique de ladite source de protéines à une température comprise entre 40 et 65°C, à un pH situé entre 6 et 9, pendant 1 à 5 heures, après l'ajout d'un mélange d'enzymes comprenant des endopeptidases dérivées de *Bacillus amyloliquefaciens* et de *Bacillus licheniformis,* de manière à obtenir un mélange réactionnel,
- l'arrêt de ladite hydrolyse enzymatique par inactivation desdites enzymes après élévation de la température dudit mélange réactionnel à un niveau non inférieur à 70°C, pendant 8 à 20 minutes.
- la séparation de l'hydrolysat de protéines obtenu du reste du mélange réactionnel.

L'hydrolyse enzymatique est réalisée à l'aide d'un mélange d'enzymes soigneusement sélectionnées pour permettre d'obtenir un hydrolysat de protéines possédant les propriétés recherchées et précitées. Le procédé, de part la nature des enzymes, la température d'hydrolyse ainsi que l'absence de solvants, respecte les qualités organoleptiques et nutritionnelles des hydrolysats obtenus. Ces hydrolysats peuvent être incorporés dans des produits alimentaires, des compositions nutraceutiques ou des préparations pharmacologiques.

Selon un mode de réalisation de l'invention, le broyage de la source de protéines est réalisé en présence d'eau selon un rapport massique source de protéines/eau de 1.

Selon un mode de réalisation de l'invention, ladite hydrolyse enzymatique est réalisée selon un ratio enzyme/source de protéines compris entre 0,01 et 2%. Préférentiellement, le ratio enzyme/source de protéines est égal à 0,5 %.

Selon un mode de réalisation de l'invention, ladite hydrolyse enzymatique est réalisée à une température de 60°C.

Selon un mode de réalisation de l'invention, ladite hydrolyse enzymatique est réalisée à un pH de 7,5.

La séparation de l'hydrolysat de protéines obtenu du reste du mélange réactionnel est généralement réalisée par centrifugation à une vitesse comprise entre 4000 et 7000 RPM et élimination du culot obtenu. Préférentiellement, la séparation de l'hydrolysat de protéines obtenu peut être réalisée par filtration dudit mélange réactionnel préalablement à ladite centrifugation. La filtration du milieu réactionnel permet d'éliminer les matières solides.

Selon un mode de réalisation de l'invention, ledit procédé comprend, en outre, la concentration et l'atomisation ou la lyophilisation dudit hydrolysat obtenu.

Selon un mode de réalisation de l'invention, l'arrêt de ladite hydrolyse enzymatique est réalisé lorsque le degré d'hydrolyse atteint une valeur maximale de 9 % et préférentiellement entre 8,75 et 8,95 %.

Selon un mode de réalisation de l'invention, le pH du mélange réactionnel au cours de l'hydrolyse est contrôlé et maintenu constant par addition d'hydroxyde de sodium à 1 mol.L$^{-1}$.

Selon un autre mode de réalisation de l'invention, ledit mélange d'enzymes comprend, de plus, une endopeptidase dérivée d'*Aspergillus oryzae.*

L'hydrolysat de protéines obtenu après réaction d'hydrolyse en présence d'un mélange de trois enzymes respectivement dérivées de *Bacillus amyloliquefaciens,* de *Bacillus licheniformis* et d'*Aspergillus oryzae* présente les mêmes propriétés et caractéristiques physico-chimiques qu'un hydrolysat de protéines obtenu après réaction d'hydrolyse en présence d'un mélange de deux enzymes respectivement dérivées de *Bacillus amyloliquefaciens* et de *Bacillus licheniformis.*

Selon un mode de réalisation avantageux du procédé selon l'invention, le mélange d'enzymes est choisi parmi le mélange CR1020 ou le mélange Protamex. Le mélange CR 1020 est commercialisé par la société Meatzyme (Chr Winthersvej 36A, 2800 Kgs Lyngby, Denmark). Le mélange Protamex est commercialisé par la société Novozyme (Krogshoejvej 36, DenmarK-2880 Bagsvaerd).

Selon un mode de réalisation de l'invention, l'arrêt de ladite hydrolyse enzymatique est réalisé par élévation de la température dudit mélange réactionnel jusqu'à 90°C et maintien de cette température pendant 10 minutes.

Selon un mode préféré de réalisation de l'invention, ledit broyage de ladite source de protéines est réalisé à partir du filet dudit, ou desdits poissons.

Le procédé selon l'invention permet ainsi l'obtention d'un hydrolysat de protéines de poisson tel que décrit précédemment.

La présente invention concerne encore une composition, un produit alimentaire ainsi qu'un complément alimentaire comprenant un hydrolysat de protéines de poisson tel que décrit précédemment.

La présente invention concerne encore un médicament comprenant un hydrolysat de protéines de poisson tel que décrit précédemment, ainsi que l'utilisation d'un tel hydrolysat de protéines de poisson pour la fabrication d'un médicament destiné au traitement de l'obésité et du diabète de type II. En effet, comme expliqué précédemment, l'hydrolysat de protéines de poisson selon l'invention peut être utilisé dans le traitement ou la prévention de telles pathologies.

La présente invention concerne encore l'utilisation non pharmaceutique d'un hydrolysat de protéines de poisson tel que défini précédemment dans l'augmentation du phénomène de la satiété.

Les formulations nutraceutiques ou pharmaceutiques incorporant un hydrolysat de protéines de poisson selon l'invention, pourront comprendre des ingrédients couramment utilisés dans ce type de formulations tels que des liants, des agents de saveurs, des conservateurs, des colorants, et pourront, dans le cas de compléments alimentaires ou de médicaments, se présenter sous la forme de comprimés, granulés ou gélules. Des formulations selon l'invention pourront également se présenter sous la forme de produits alimentaires tels que des boissons, ou encore sous la forme de suspensions ou de sirops.

Les caractéristiques de l'invention mentionnées ci-dessus, ainsi que d'autres, apparaîtront plus clairement à la lecture de la description suivante d'un exemple de réalisation, ledit exemple se voulant illustratif et non limitatif.

La Fig. 1 illustre l'évolution du degré d'hydrolyse de l'hydrolysat de protéines de merlan bleu selon l'invention,

La Fig. 2 illustre la répartition des poids moléculaires des fragments protéiques d'un hydrolysat de protéine de merlan bleu selon l'invention, et

Les Figs. 3 à 5 illustrent les répartitions des poids moléculaires des fragments protéiques d'hydrolysats de protéine d'autres espèces de poisson selon l'invention,

La Fig. 6 illustre la sécrétion de molécules de CCK par les cellules STC-1 en présence ou en l'absence d'un hydrolysat de protéine de merlan bleu selon l'invention,

La Fig. 7 illustre la sécrétion de molécules de GLP1 par les cellules STC-1 en présence ou en l'absence d'un hydrolysat de protéine de merlan bleu selon l'invention,

La Fig. 8 illustre un effet d'un hydrolysat de protéine de merlan bleu selon l'invention sur la prise alimentaire chez le rat, et

Les Figs. 9 et 10 représentent les dosages plasmatiques de molécules de CCK et de GLP1, respectivement, chez le rat après la prise ou non d'un hydrolysat de protéine de merlan bleu selon l'invention.

**Exemple 1: Hydrolysat de protéines obtenu à partir de merlan bleu, *Micromesistius poutassou* (H1)**

Le merlan bleu *(Micromesistius poutassou)* est pêché en Atlantique Nord au large de Terre-Neuve. Les poissons sont débités en filets lesquels sont ensuite broyés de manière à en obtenir la pulpe. Cette pulpe de poisson constitue une source de protéines pour la production de l'hydrolysat. La pulpe est conservée à -20°C jusqu'à utilisation.

Trois kilogrammes de pulpe de merlan bleu préalablement décongelés sont mélangés à de l'eau selon un rapport massique de 1. La température du mélange est portée à 60°C et le pH est ajusté à la valeur de 7,5 au moyen d'une solution d'hydroxyde de sodium à 1 M, sous agitation.

Un mélange composé de trois enzymes, respectivement dérivées de *Bacillus amyloliquefaciens,* de *Bacillus licheniformis* et *d'Aspergillus oryzae,* et commercialisé sous le nom CR 1020 par la société Meatzyme (Chr Winthersvej 36A, 2800 Kgs Lyngby, Denmark) est alors ajouté dans le mélange réactionnel selon un ratio enzyme/source de protéines de 0,5 %. Durant la réaction d'hydrolyse, le pH est maintenu constant à une valeur de 7,5 par addition d'hydroxyde de sodium (NaOH) à 1M.

La réaction d'hydrolyse des protéines de merlan bleu est menée pendant 2 heures en conditions contrôlées grâce à la méthode bien connue dite pH-STAT. La méthode du pH-STAT est basée sur le maintien constant du pH pendant la réaction d'hydrolyse. L'étendue de l'hydrolyse est ainsi quantifiée par le degré d'hydrolyse (DH) qui est déterminé par le nombre de liaisons peptidiques coupées sur le nombre total de liaisons peptidiques.

Le DH est calculé à partir du volume et de la molarité de la base utilisée pour maintenir le pH constant. Tant que le pH reste constant, il existe une relation entre le nombre de liaisons hydrolysées et le volume d'hydroxyde de sodium versé. Pour un système enzymatique donné et à pH constant, la fonctionnalité sera la même d'un hydrolysat à un autre, si la réaction est stoppée chaque fois au même DH.

$$\% \, DH = [(B. \, NB)/(\alpha \, . \, htot \, . \, MP)] * 100$$

Avec :

- B la consommation de la base (en ml ou L)
- NB la normalité de la base
- $\alpha$ la moyenne de dissociation des groupes HN ou COOH
- MP la masse de protéines (déterminée par la méthode de Kjeldhal, en g ou kg)
- htot le nombre total de liens peptidiques

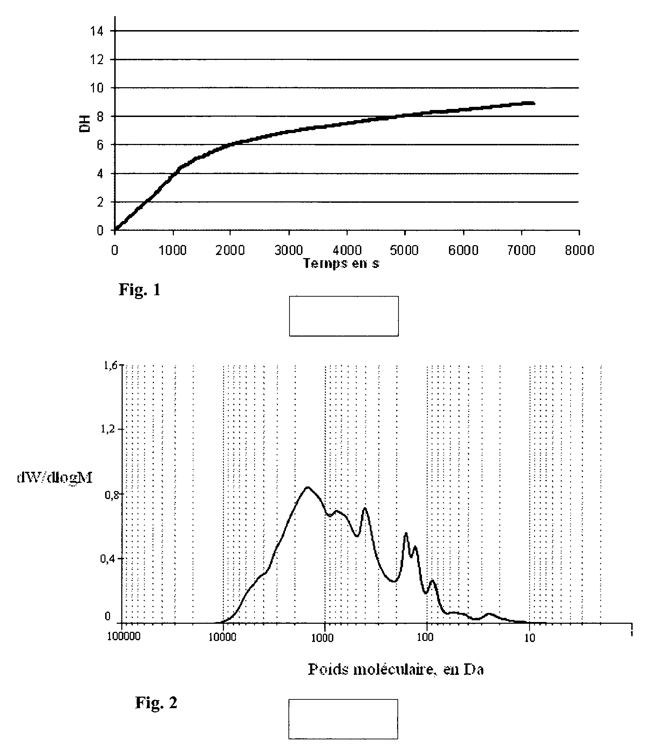

Après 2 heures de réaction d'hydrolyse, le DH final de l'hydrolysat de protéines est de 8.9 % (Fig. 1).

L'inactivation des enzymes au terme de la cinétique d'hydrolyse est réalisée par élévation de la température du milieu réactionnel jusqu'à 90°C. Cette température est maintenue durant 10 minutes.

L'hydrolysat de protéines de merlan bleu obtenu, dénommé par la suite H1, est ensuite filtré sur un tamis (grillage de 2 mm/2 mm) de manière à éliminer les matières solides. La fraction récupérée dans le récipient est alors centrifugée pendant 30 minutes +/- 5 minutes, à une vitesse comprise entre 4000 et 7000 RPM. Après élimination du culot, le surnageant est récupéré, lyophilisé et conservé dans un endroit frais et sec, à l'abri de la lumière. Le surnageant peut également être atomisé.

Dans une variante de l'invention, il est possible de désactiver les enzymes endogènes par élévation de la température jusqu'à l'ébullition préalablement à l'ajout du mélange des enzymes précitées.

Dans une autre variante de l'invention, l'hydrolyse enzymatique est réalisée à partir d'un mélange composé de deux enzymes respectivement dérivées de *Bacillus amyloliquefaciens* et de *Bacillus licheniformis* et commercialisé sous le nom Protamex par la société Novozyme (Krogshoejvej 36, Denmark-2880 Bagsvaerd).

Analyses physico-chimiques de l'hydrolysat de protéines obtenu à partir de merlan bleu.

Une détermination des poids moléculaires des peptides constitutifs de l'hydrolysat de protéines obtenu est réalisée par chromatographie d'exclusion stérique (SEC-HPLC).

L'hydrolysat de protéines H1, sous forme de poudre après lyophilisation est suspendu dans de l'eau ultra pure (20 mg/mL), puis filtré sur une membrane de 0,45 $\mu$m et analysé par filtration sur gel avec une colonne Superdex Peptide HR 10/30, commercialisé par la société Pharmacia. La matrice de la colonne est composée d'un gel poreux réticulé (diamètre 13-15$\mu$m) d'agarose et de dextran d'un volume total de 24 mL. Son domaine de fractionnement est compris entre 100 et 7000 Da. La colonne est montée sur une chaîne HPLC (commercialisée par la société Dionex) qui est équipée d'une pompe (module Dionex P680). La mesure est réalisée par un détecteur d'Ultra Violet multi longueur d'onde (module Dionex UVD 170 U). L'hydrolysat de protéines est élué par une phase mobile contenant de l'acétonitrile, de l'eau et du TFA. L'élution dure environ 1 heure à un débit de 0,5mL/min.

La répartition des poids moléculaires est calculée à partir des paramètres d'une droite de calibration obtenue après le passage dans la colonne de marqueurs de poids moléculaires connus. Ces marqueurs sont le cytochrome C (12 400 Da), l'aprotinine (6511 Da), la gastrine 1 (2 126 Da), la substance P (1 348 Da), la substance P fragment 1-7 (900Da), la glycine (75 Da) et la leupeptine (463 Da). Les données sont collectées grâce au logiciel Chromeleon (Dionex). Les pourcentages des poids moléculaires sont calculés à l'aide d'un logiciel (GPC Cirrus de chez Polymer Laboratories). La longueur d'onde d'acquisition est de 214 nm. La répartition des poids moléculaires en fonction dW/logM est donnée sur la Fig. 2, et la répartition des poids moléculaires par classe de taille est donnée dans le Tableau 2, *infra.* Le pourcentage de l'aire sous la courbe correspond au pourcentage de molécules peptidiques.

La composition en acides aminés de l'hydrolysat de protéines de merlan bleu H 1 est donnée dans le Tableau 1 (selon directive européenne 98/64/CE et NF EN ISO 13904-Octobre 2005). Le Tableau 2 représente la répartition des acides aminés dans l'hydrolysat de protéines H1.

Tableau 1

| Acide aminé | Pourcentage d'acide aminé | Acide aminé | Pourcentage d'acide aminé |
|---|---|---|---|
| Acide glutamique | 17,4 | Glycine | 5 |
| Lysine | 10,2 | Thréonine | 4,5 |
| Acide aspartique | 11,4 | Sérine | 4,4 |
| Leucine | 8,4 | Tyrosine | 3,2 |
| Arginine | 6,1 | Phénylalanine | 3,9 |
| Alanine | 6,8 | Méthionine | 2,5 |
| Valine | 4,7 | Proline | 3,6 |
| Isoleucine | 4,2 | Histidine | 1,9 |
| Cystine | 1 | Tryptophane | 0,8 |

La teneur en protéines est supérieure à 80 %, en pourcentage de produit brut (selon NF V18-120-Mars 1997-corrigé KJELDAHL).

La teneur en lipides est inférieure à 1 %, en pourcentage de produit brut (selon directive européenne 98/64/CE).

La valeur énergétique de l'hydrolysat de protéines H1 est d'environ 330 Kcal/100g.

La teneur en glucides est inférieure à 0,1 % (déduite à partir des teneurs en protéines et glucides et de la valeur énergétique).

**Exemple 2: Hydrolysat de protéines obtenu à partir d'autres espèces de poisson selon l'invention**

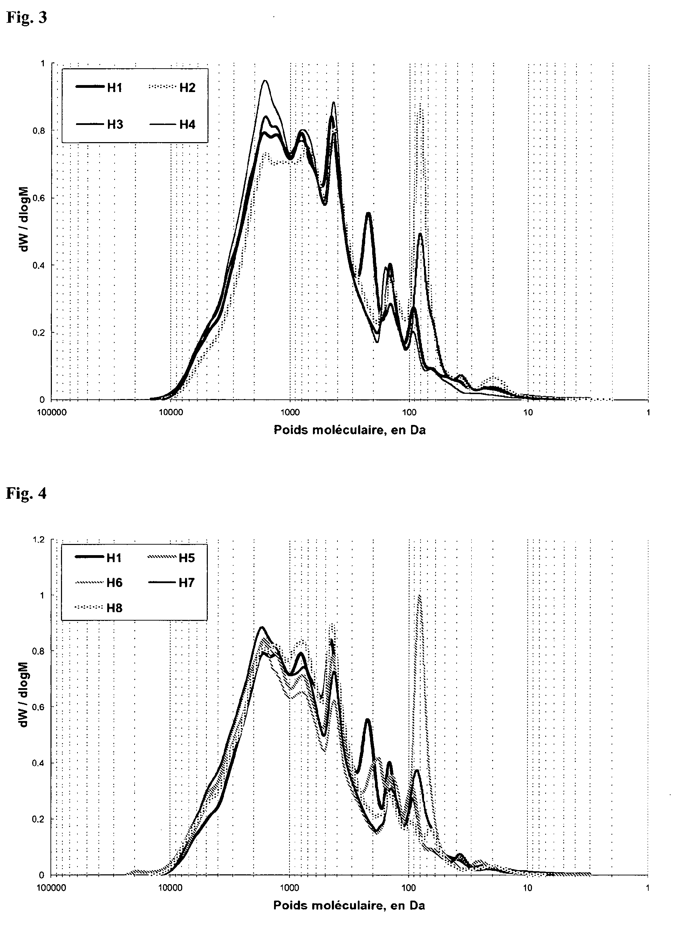

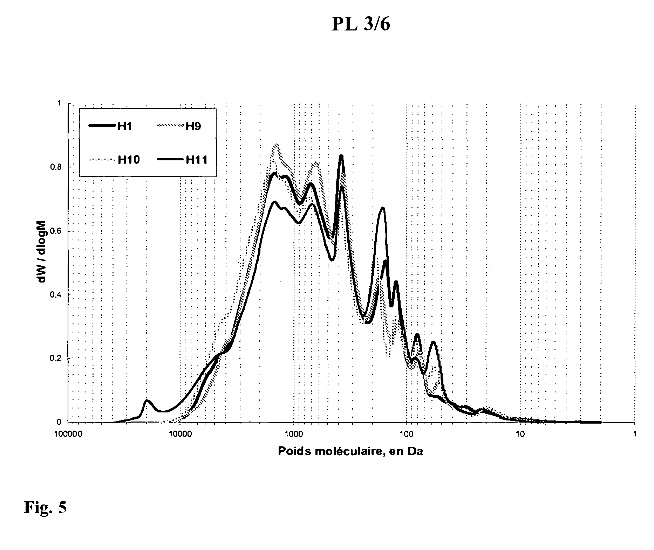

Des hydrolysats de protéines de maquereau (H2) (scomber scombrus), chinchard (H3) (Trachurus spp.), grenadier (H4) (*Coryphaenoides rupestris*) (Fig. 3); tacaud (H5) (*Trisopterus esmarki),* sardine (H6) (*Sardina pilchardus),* hareng (H7) (*Clupea harengus*), panga (H8) (Suliforme) (Fig. 4); cabillaud (H9) (*Gadus morhua*), lieu noir (H:10) (*Pollachius virens*) et églefin (H11) (*Melanogrammus aeglefinus*) (Fig. 5) ont été préparés selon le procédé de l'exemple 1. La répartition des poids moléculaires des peptides composant chaque hydrolysat a été analysée selon le même procédé que celui utilisé dans l'exemple 1.

La répartition des poids moléculaires en fonction dW/logM est donnée sur les Figs. 3 à 5, et la répartition des poids moléculaires par classe de taille est donnée dans le Tableau 2 suivant. Le pourcentage de l'aire sous la courbe correspond au pourcentage de molécules peptidiques.

Tableau 2

| Classes | H1 | H2 | H3 | H4 | H5 | H6 | H7 | H8 | H10 | H9 | H11 |
|---|---|---|---|---|---|---|---|---|---|---|---|
| <0,3 | 23-31 | 30 | 23 | 23 | 23 | 25 | 23 | 23 | 24 | 24 | 29 |
| 0,3-1 | 31-34 | 33 | 34 | 33 | 32 | 31 | 32 | 33 | 31 | 34 | 33 |
| 1-3 | 28-34 | 29 | 33 | 34 | 33 | 32 | 33 | 34 | 33 | 34 | 28 |
| 3-5 | 6-8 | 6 | 7 | 7 | 8 | 8 | 8 | 7 | 8 | 6 | 6 |
| 5->10 | 2-4 | 2 | 3 | 3 | 4 | 4 | 4 | 3 | 4 | 2 | 4 |

Les hydrolysats H1 à H11 présentent un profile de répartition des poids moléculaires identique.

<u>Exemple 3: Dosage de molécules immunologiquement apparentées aux cholecystokinines (CCK) dans l'hydrolysat de protéines de merlan bleu H1</u>

Les molécules apparentées aux CCK présentes dans l'hydrolysat de protéines H1 ont été dosées par dosage radio immunologique à l'aide du kit RIA (GASK-PR, CIS bio international, Bagnols/Cèze, France) (test réalisé en triplicat). On entend par molécules apparentées aux CCK, toutes molécules capables de se fixer à un anticorps spécifique dirigé contre les huit acides aminés communs aux gastrines et CCK, le dit anticorps étant l'anticorps utilisé dans le dosage cité ci-dessus. La gastrine et les CCK possèdent des séquences peptidiques identiques dans la partie C-terminale de leurs séquences peptidiques.

L'hydrolysat de protéines contient 5,6 pg de molécules apparentées aux gastrines/CCK par mg de poids sec d'hydrolysat.

L'hydrolysat selon l'invention permet ainsi un apport de molécules apparentées aux molécules de CCK.

<u>Exemple 4: Test de culture cellulaire *in vitro:* effet sur la stimulation de la sécrétion de molécules de CCK et sur la stimulation de la sécrétion de molécules de GLP 1</u>

L'hydrolysat de protéines H1 a été testé pour sa capacité à stimuler la sécrétion de molécules de CCK, ainsi que de GLP1, au niveau de cellules entéroendocrines de type STC1. En effet, la sécrétion de CCK et de GLP1 par les cellules endocrines intestinales représente l'un des principaux signaux constituant le phénomène de satiété. Les cellules STC-1 sont des cellules pluri hormonales dérivées de cellules endocrines tumorales issues de l'intestin grêle d'une souris. Les cellules STC1 sont utilisées comme modèle cellulaire pour l'étude des phénomènes induisant la sécrétion spécifique de CCK [10] ainsi que comme modèle cellulaire pour l'étude des phénomènes induisant la sécrétion spécifique de GLP1 [11].

Les cellules STC-1 ont été cultivées dans du milieu DMEM contenant 2mM de L-glutamine, 2mM de pénicilline, 50 $\mu$M de streptomycine, et 10% de sérum de veau foetal (SVF). Entre 2 et 3 jours avant la réalisation du test, les cellules STC-1 ont été mises en cultures dans des plaques de 24 puits à hauteur de 30 000 à 40 000 cellules par puits. Lorsque les cellules eurent atteint un niveau de confluence d'environ 85%, les puits ont été rincés deux fois avec du tampon d'incubation (4,5 mM KCl, 1,2mM $CaCl_2$, 1.2 mM $MgCl_2$, 10 mM Glucose, 140 mM NaCl et 20 mM Hepes-Tris, pH 7,4).

Les cellules ont ensuite été incubées durant 2 heures en présence de différentes solutions composées soit d'albumine sérique bovine (BSA), soit de l'hydrolysat de protéines H1 à différentes concentrations, soit d'acides aminés libres (cf. Tableau 3), soit d'un hydrolysat commercial d'albumine d'oeuf (AEH) ou du tampon d'incubation utilisé comme témoin de culture (contrôle). Les surnageants des cultures ont été centrifugés (5 min, 2000g). Après centrifugation, les surnageants ont été récupérés et conservés à -20°C avant d'en doser le contenu en CCK par dosage radio immunologique grâce au kit RIA (GASK-PR, CIS Bio International, Bagnols/Cèze, France) (les cellules STC-1 ne sécrètent pas de gastrines). Le dosage est réalisé suivant le protocole fourni par le distributeur. Les concentrations de GLP-1 sous sa forme active sécrétée par les cellules STC-1, ont été déterminées grâce au kit de dosage radioimmunologique (GLP1A-35HK, Linco Research, St Charles, MO, USA). Le dosage est réalisé suivant le protocole fourni par le distributeur.

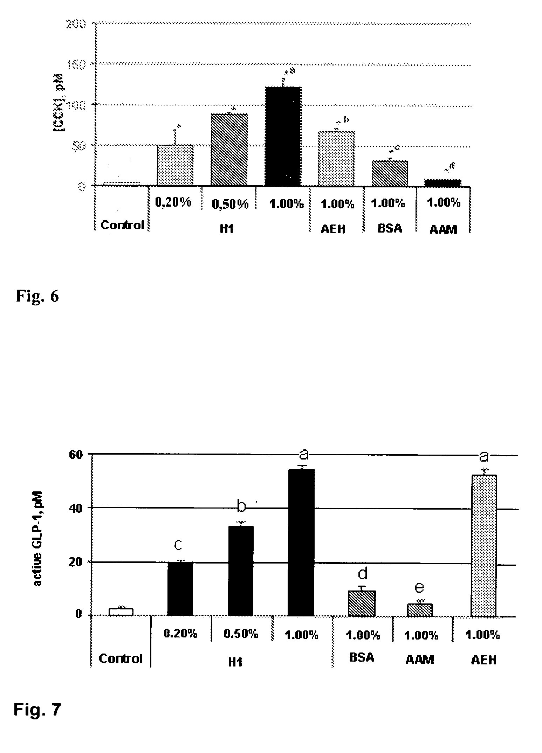

Les résultats concernant le dosage des molécules de CCK sont présentés sur la Fig. 6 et sont exprimés en picomoles/L (pM) de CCK excrétées par les cellules. Les résultats concernant le dosage des molécules de GLP1 sont présentés sur la Fig. 7 et sont exprimés en picomoles/L (pM) de GLP1 excrétées par les cellules.

Sur les Figs. 6 et 7, le signe * désigne des valeurs significativement différentes de la valeur obtenue pour le contrôle (t-test ($P < 0,05$)). Les lettres (a, b, c, d) représentent des valeurs significativement différentes les unes des autres (t-test, $P<0,05$).

Tableau 3 : Composition de la solution en acides aminés libres

| Acide aminé | Quantité (mg/L) | Acide aminé | Quantité (mn/L) |
|---|---|---|---|
| Acide L-glutamique | 151 | Glycine | 128 |
| L-Lysine | 88 | L-Thréonine | 64 |
| Acide L-aspartique | 151 | L-Sérine | 26 |
| L-Leucine | 112 | L-Tyrosine | 06 |
| L-Arginine | 277 | L-Phénylalanine | 91 |
| L-Alanine | 96 | L-Méthionine | 40 |
| L-Valine | 80 | L-Proline | 53 |

(suite)

| Acide aminé | Quantité (mg/L) | Acide aminé | Quantité (mn/L) |
|---|---|---|---|
| L-Isoleucine | 64 | L-Histidine | 72 |
| L-Cystéine | 16 | L-Tryptophane | 16 |

Les résultats montrent l'effet significatif de l'hydrolysat de protéines de merlan bleu H1 à différentes concentrations (0,2 ; 0,5 et 1,0 %, rapport masse/volume), sur la sécrétion à la fois de molécules CCK et de molécules de GLP1 dans le milieu extracellulaire par les cellules STC-1 par rapport aux autres solutions testées.

Les quantités de molécules CCK et GLP1 libérées par les cellules ont augmenté de manière significative avec les concentrations de l'hydrolysat (Fig. 6 et 7). La quantité de molécules de CCK obtenue à partir de l'hydrolysat concentré à 1,0 % fut 30 fois supérieure à celle obtenue avec le témoin de culture (122,03 pM de CCK contre 4,02 pM de CCK, respectivement). Les quantités de CCK obtenues en présence de BSA ou d'acides aminés libres à 1.0 % sont considérablement inférieures à celles obtenues en présence des hydrolysats à la même concentration (31,2 et 8,6 pM, respectivement). Les mêmes observations sont constatées en ce qui concerne la sécrétion des molécules de GLP1.

Ces résultats indiquent que l'hydrolysat de protéines de merlan bleu contient des molécules capables de stimuler fortement la sécrétion des molécules de CCK et de GLP1 par les cellules STC-1. Les faibles potentiels stimulants des solutions de BSA et d'acides aminés libres indiquent que l'effet stimulateur de la sécrétion des molécules de CCK et GLP1 n'est dû ni d'une part à un « effet protéine », ni d'autre part à l'action des acides aminés libres présents en grand nombre dans l'hydrolysat de merlan bleu. Cette stimulation apparaît donc comme étant majoritairement due aux molécules peptidiques contenues dans l'hydrolysat de protéines H1.

**Exemple 5 : Mise en évidence de l'effet satiétogène d'un hydrolysat de protéine selon l'invention chez le rat - Etude *in vivo-***

Les propriétés de l'hydrolysat H1 obtenu selon l'exemple 1 ont été évaluées sur la prise alimentaire chez le rat, ainsi que sur différents paramètres sanguins. Le but étant de mettre en évidence un effet satiétogène de l'hydrolysat sur la prise alimentaire, corroboré par des paramètres physiologiques endocriniens.

*Protocole expérimental :*

32 rats Wistar mâles de 275 à 290 grammes (Harlan, France) divisés en 4 groupes de 8 individus, sont maintenus dans des cages individuelles opaques fermées par une grille en aluminium (pour les priver de la vue des autres rats et ne pas perturber leur alimentation). Ils sont installés dans une salle climatisée à une température de $21 \pm 1°C$ et un cycle jour : nuit de 12 heures (17h/5h). Ils ont un accès *ad libitum* à la nourriture (Aliment complet, Harlan, France) et à l'eau durant la période d'adaptation (5 jours) et les deux premières semaines d'expérimentation.

Chaque groupe de rat est gavé avec des compositions de gavage différentes :

- groupe 1 : eau
- groupe 2 : H1 (50 mg.jour$^{-1}$)
- groupe 3 : H1 (100 mg.jour$^{-1}$)
- groupe 4 : H1 (250 mg.jour$^{-1}$)

Chaque rat est gavé de manière individuelle dans une salle à part, hors de la vue des autres rats, par 0.5 ml d'eau ou d'hydrolysat H1 dissous (50, 100 ou 250 mg.mL$^{-1}$ selon le groupe). La durée du gavage peut être estimée à trois minutes par rat et permet d'effectuer la mesure de la prise alimentaire (par pesée de l'aliment consommé) en parallèle, l'aliment complet est présenté au rat 10 minutes après le gavage. Les litières sont également changées au moment du gavage une fois par semaine et la pesée des rats a lieu une fois par semaine le lundi.

**1. Prise alimentaire**

Après avoir été pesés afin de former des groupes de poids moyens équivalents, les rats sont maintenus en période d'adaptation durant les sept jours précédant le début de l'expérimentation. La phase d'expérimentation débute le lundi (J1) de la première semaine et se poursuit durant deux semaines.

Le premier jour de l'expérimentation (J1) au matin, les rats sont mis à jeun durant 24 heures. Le second jour (J2) au matin, les rats sont pesés et subissent un prélèvement sanguin au niveau de l'extrémité de la queue (collecte dans tubes contenant de l'EDTA à 5%) à la suite duquel l'aliment complet leur est remis à disposition. Les échantillons sanguins sont ensuite centrifugés, le plasma est conservé à -20°C.

Les rats des 4 groupes sont gavés une première fois par leur composition de gavage respective par voie orogastrique à l'aide d'une sonde intragastrique le second jour (J2) à 17h. Une première mesure de la prise alimentaire par pesée a lieu 2h plus tard. Le matin du troisième jour (J3), la prise alimentaire est à nouveau évaluée avant le gavage réalisé à 9h puis une mesure de la prise alimentaire est de nouveau réalisée 3h plus tard. Les rats sont à nouveau gavés à 17h et dans le même temps la prise alimentaire est mesurée, puis à nouveau mesurée 2h plus tard. Ces étapes de gavages et de mesures sont réalisées jusqu'au cinquième jour (J5) au soir inclus, et répétées une seconde semaine du lundi (jour J8) au matin au vendredi (jour J12) au soir.

## 2. Hormones plasmatiques

A la fin de la deuxième semaine, les rats sont sacrifiés par décapitation 30 minutes après le gavage et le sang est prélevé sur tube/EDTA à 5%. 3 aliquotes de plasma seront réalisés afin de doser les hormones circulantes suivantes :

- aliquote 1 : GLP-1
- aliquote 2 : CCK

## *Résultats*

### 1. Prise alimentaire

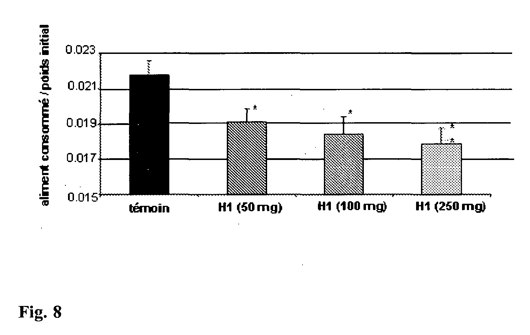

Les résultats présentés dans la Fig. 8 expriment la consommation alimentaire ajoutée des rats, en gramme d'aliment et en fonction de leur poids initial, au cours de l'ensemble des deux semaines d'expérimentation. Les valeurs sont les moyennes des valeurs journalières obtenues pour chaque groupe au cours de l'expérimentation, et exprimées $\pm$ SEM ; * : $p < 0.05$, ** $p < 0.01$.

Ainsi les résultats montrent qu'entre 9h et 19h les valeurs relatives à la prise alimentaire obtenues pour les groupes 2, 3 et 4 ayant reçu H1 sont significativement inférieures à celle obtenues pour le groupe témoin 1 qui n'a pas reçu H1. Une diminution de la prise alimentaire en fonction de la dose d'hydrolysat H1 journalière est observée ; bien qu'il n'existe pas de différence significative entre les groupes 2, 3 et 4, la probabilité qu'il existe une différence entre le groupe témoin 1 et les autres groupes 2, 3, 4 augmente avec la dose journalière d'hydrolysat administrée.

### 2. Hormones plasmatiques

Les concentrations en CCK dans les plasmas de rats ont été mesurées à l'aide d'un dosage radioimmunologique mis au point par la Compagnie des Pêches Saint Malo-Santé. Ce dosage a la particularité d'utiliser un anticorps spécifique, développé en 1998 (Rehfeld 1998), des CCK actives sulfatés, qui ne croisent pas avec les différentes formes de gastrine (présentes dans le plasma en plus grande quantité que les CCK). Ce protocole a été mis au point notamment à partir d'un kit de dosage distribué par la société IBL ((IBL, Hamburg, Germany) utilisant le même anticorps.

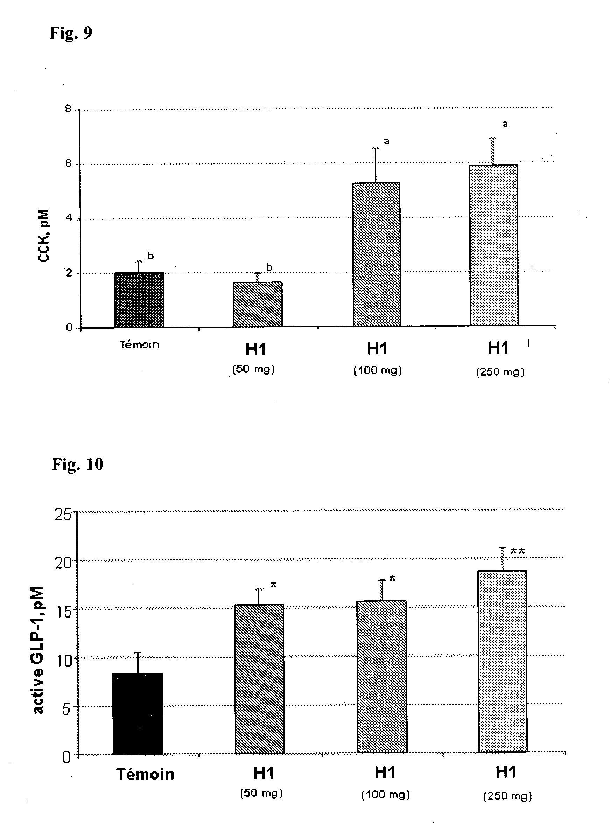

Les concentrations de GLP-1 sous sa forme active plasmatique ont été déterminées grâce au kit de dosage radioimmunologique (GLP1A-35HK, Linco Research, St Charles, MO, USA). Le dosage est réalisé suivant le protocole fourni par le distributeur. Les résultats sont présentés sur les Figs. 9 et 10. Les concentrations plasmatiques de GLP-1 (Fig. 10) et CCK (Fig. 9) sont exprimées en $pmol.L^{-1}$ de plasma sanguin. Les valeurs sont les moyennes de chaque groupe, exprimées $\pm$ SEM. * : T-test, $p<0.05$, ** : T-test, $p<0.01$. Sur la Fig. 9, les moyennes n'étant pas assignées d'une lettre identique sont différentes.

On remarque que les concentrations plasmatiques en GLP1 sont significativement différentes de celle obtenue avec le témoin, quelle que soit la dose d'hydrolysat reçue par l'animal. Les concentrations plasmatiques en CCK pour les groupes ayant reçu 100 ou 250 $mg.jour^{-1}$ de H1 sont significativement différentes de celle obtenue avec le témoin. Cette analyse rejoint l'analyse de la prise alimentaire.

BIBLIOGRAPHIE

[1] Strader AD, Woods SC. Gastrointestinal hormones and food intake. Gastroenterology 2005; 128:175-191
[2] Moran TH, Kinzig KP. Gastrointestinal satiety signals II. Cholecystokinin. Am. J. Physiol. Gastrointest. Liver Physiol. 2004; 286 : G183-188.
[3] Moran TH. Cholecystokinin and satiety: current perspectives. Nutrition 2000; 16:858-865

[4] Chaudhri O, Small C, Bloom S. Gastrointestinal hormones regulating appetite. Philos Trans R Soc Lond B Biol Sci 2006; 361:1187-209.

[5] Cuber JC, Bernard C, Levenez F, Chayvialle JA. [Lipids, proteins and carbohydrates stimulate the sécrétion of intestinal cholecystokinin in the pig]. Reprod Nutr Dev 1990; 30:267-75.

[6] Baile CA, McLaughlin CL, Della-Fera MA. Role of cholecystokinin and opioid peptides in control of food intake. Physiol Rev 1986; 66 : 172-234.

[7] Liddle RA, Green GM, Conrad CK, Williams JA. Proteins but not amino acids, carbohydrates, or fats stimulate cholecystokinin secretion in the rat. Am J Physiol 1986; 251:G243-8.

[8] Douglas BR, Woutersen RA, Jansen JB et al. The influence of different nutrients on plasma cholecystokinin levels in the rat. Experientia 1988; 44:21-3.

[9] Miazza B, Palma R, Lachance JR et al. Jejunal secretory effect of intraduodenal food in humans. A comparison of mixed nutrients, proteins, lipids, and carbohydrates. Gastroenterology 1985; 88:1215-22.

[10] Mangel AW et al. Phenylalanine-stimulated sécrétion of cholecystokinin is calcium dependent. Am J Physiol, 1995. 268(1 Pt 1): p. G90-4.

[11] Brubaker PL, Izzo A, Rocca AS. Synthesis and secretion of intestinal proglucagon-derived peptides by the STC-1 enteroendocrine cell line. Can J Diabetes. 2003;27:141-148.

## Revendications

1. Hydrolysat de protéines de poisson **caractérisé en ce qu'**il est obtenu par hydrolyse enzymatique d'au moins une source de protéines choisie parmi le groupe composé des espèces de poissons *Micromesistius poutassou, Clupea harengus, Scomber scombrus, Sardina pilchardus, Gadus morhua, Pollachius virens, Melanogrammus aeglefinus, Coryphaenoides rupestris, Trisopterus esmarki, Trachurus spp.,* poissons appartenant à l'ordre des siluriformes, ladite hydrolyse enzymatique étant réalisée par un mélange d'enzymes comprenant des endopeptidases dérivées de *Bacillus amyloliquefaciens* et de *Bacillus licheniformis* et **en ce qu'**il présente:

   - la répartition du profil moléculaire suivant : de 23 à 31 % de molécules de poids moléculaire inférieur à 300 Da, de 31 à 34 % de molécules dont le poids moléculaire est compris entre 300 et 1 000 Da, de 28 à 34 % de molécules dont le poids moléculaire est compris entre 1 000 et 3 000 Da, de 6 à 8 % de molécules dont le poids moléculaire est compris entre 3 000 et 5 000 Da et de 2 à 4 % de molécules dont le poids moléculaire est compris entre 5 000 et 10 000 Da,
   - une teneur en lipides inférieure à 1 %, en pourcentage de produit brut,
   - une teneur en glucides inférieure à 0,1 %, en pourcentage de produit brut,
   - une teneur en protéines supérieure à 80 %, en pourcentage de produit brut,
   - une teneur en matière minérale comprise entre 10 et 20 %, en pourcentage de produit brut,

   et **en ce qu'**il comprend des molécules immunologiquement apparentées aux cholecystokinines (CCK).

2. Hydrolysat de protéines de poisson selon la revendication 1, **caractérisé en ce qu'**il présente la composition en acides aminés suivante : Acide glutamique 17,4 %, Acide aspartique 11,4 %, Lysine 10,2 %, Leucine 8,4 %, Arginine 6,1 %, Alanine 6,8 %, Valine 4,7 %, Isoleucine 4,2 %, Glycine 5 %, Thréonine 4,5 %, Sérine 4,4 %, Tyrosine 3,2 %, Phénylalanine 3,9 %, Méthionine 2,5 %, Proline 3,6 %, Histidine 1,9 %, Cystine 1 %, Tryptophane 0,8 %, en pourcentage en poids par rapport au poids total d'acides aminés.

3. Hydrolysat de protéines de poisson selon l'une des revendications 1 à 2, **caractérisé en ce que** ladite source de protéines de poisson comprend la pulpe obtenue à partir du filet dudit ou desdits poissons.

4. Hydrolysat de protéines de poisson selon l'une des revendications 1 à 3, **caractérisé en ce que** ledit mélange d'enzymes comprend, de plus, une endopeptidase dérivée *d'Aspergillus oryzae.*

5. Procédé d'obtention d'un hydrolysat de protéines de poissons tel que défini dans les revendications 1 à 4, **caractérisé en ce qu'**il comprend:

   - le broyage d'au moins une source de protéines choisie parmi le groupe composé des espèces de poissons *Micromesistius poutassou, Clupea harengus, Scomber scombrus, Sardina pilchardus, Gadus morhua, Pollachius virens, Melanogrammus aeglefinus, Coryphaenoides rupestris, Trisopterus esmarki, Trachurus spp.,* poissons appartenant à l'ordre des siluriformes, en présence d'eau, de manière à récupérer la pulpe dudit ou desdits poissons,

- l'hydrolyse enzymatique de ladite source de protéines à une température comprise entre 40 et 65°C, à un pH compris entre 6 et 9, pendant 1 à 5 heures, après l'ajout d'un mélange d'enzymes comprenant des endopeptidases dérivées de *Bacillus amyloliquefaciens* et de *Bacillus licheniformis,* selon un ratio enzyme/source de protéines compris entre 0,01 et 2%, de manière à obtenir un mélange réactionnel,

- l'arrêt de ladite hydrolyse enzymatique par inactivation desdites enzymes après élévation de la température dudit mélange réactionnel à un niveau non inférieur à 70°C, pendant 8 à 20 minutes.

- la séparation de l'hydrolysat de protéines obtenu du reste du mélange réactionnel.

6. Procédé selon a revendication 5, **caractérisé en ce que** ledit ratio enzyme/source de protéines est égal à 0,5 %, ladite température d'hydrolyse est égale à 60°C et ledit pH est de 7,5.

7. Procédé selon l'une des revendications 5 à 6, **caractérisé en ce que** ledit arrêt de ladite hydrolyse enzymatique est réalisé lorsque le degré d'hydrolyse atteint 8.9.

8. Procédé selon l'une des revendications 5 à 7, **caractérisé en ce que** ledit mélange d'enzymes contient, de plus, une endopeptidase dérivée *d'Aspergillus oryzae.*

9. Composition **caractérisée en ce qu'**elle comprend un hydrolysat de protéines de poissons tel que défini dans les revendications 1 à 4.

10. Composition selon la revendication 9, **caractérisée en ce qu'**elle se présente sous la forme d'un produit alimentaire, d'un complément alimentaire ou d'une composition nutraceutique.

11. Composition pharmaceutique **caractérisée en ce qu'**elle comprend un hydrolysat de protéines de poissons tel que défini dans les revendications 1 à 4.

12. Utilisation d'un hydrolysat de protéines de poisson tel que défini dans les revendications 1 à 4 pour la fabrication d'un médicament destiné au traitement de l'obésité ou du diabète de type II.

13. Hydrolysat de protéines de poisson tel que défini dans les revendications 1 à 4 pour son utilisation dans le traitement de l'obésité ou du diabète de type II.

14. Utilisation non pharmaceutique d'un hydrolysat de protéines de poisson tel que défini dans les revendications 1 à 4 dans l'augmentation du phénomène de la satiété.


**Patentansprüche**

1. Fischproteinhydrolysat, **dadurch gekennzeichnet, dass** es durch enzymatische Hydrolyse mindestens einer Proteinquelle, ausgewählt aus der Gruppe bestehend aus den Fischarten *Micromesistius poutassou, Clupea harengus, Scomber scombrus, Sardina pilchardus, Gadus morhua, Pollachius virens, Melanogrammus aeglefinus, Coryphaenoides rupestris, Trisopterus esmarki, Trachurus* spp., Fischen, die zur Ordnung der Siluriformes gehören, erhalten wird, wobei die enzymatische Hydrolyse durch ein Enzymgemisch umfassend von *Bacillus amyloliquefaciens* und *Bacillus licheniformis* stammende Endopeptidasen durchgeführt wird, und dadurch, dass es aufweist:

- die folgende Molekulargewichtsverteilung: von 23 bis 31% an Molekülen mit einem Molekulargewicht von kleiner als 300 Da, von 31 bis 34% an Molekülen, deren Molekulargewicht zwischen 300 und 1.000 Da ist, von 28 bis 34% an Molekülen, deren Molekulargewicht zwischen 1.000 und 3.000 Da ist, von 6 bis 8% an Molekülen, deren Molekulargewicht zwischen 3.000 und 5.000 Da ist und von 2 bis 4% an Molekülen, deren Molekulargewicht zwischen 5.000 und 10.000 Da ist,

- einen Lipidgehalt von kleiner als 1%, als Prozentsatz des Rohprodukts,

- einen Kohlenhydratanteil von kleiner als 0,1%, als Prozentsatz des Rohprodukts,

- einen Proteingehalt von größer als 80%, als Prozentsatz des Rohprodukts,

- einen Mineralstoffgehalt zwischen 10 und 20%, als Prozentsatz des Rohprodukts, und dadurch, dass es zu Cholecystokininen (CCK) immunologisch ähnliche Moleküle umfasst.

2. Fischproteinhydrolysat gemäß Anspruch 1, **dadurch gekennzeichnet, dass** es die folgende Aminosäurezusammensetzung aufweist: Glutaminsäure 17,4%, Asparaginsäure 11,4%, Lysin 10,2%, Leucin 8,4%, Arginin 6,1%, Alanin 6,8%, Valin 4,7%, Isoleucin 4,2%, Glycin 5%, Threonin 4,5%, Serin 4,4%, Tyrosin 3,2%, Phenylalanin 3,9%, Methionin 2,5%, Prolin 3,6%, Histidin 1,9%, Cystin 1%, Tryptophan 0,8%, als Gewichtsprozent bezogen auf das Gesamtgewicht an Aminosäuren.

3.  Fischproteinhydrolysat gemäß einem der Ansprüche 1 bis 2, **dadurch gekennzeichnet, dass** die Fischproteinquelle von dem Filet des oder der Fischs/Fische erhaltene Pulpe enthält.

4.  Fischproteinhydrolysat gemäß einem der Ansprüche 1 bis 3, **dadurch gekennzeichnet, dass** das Enzymgemisch weiterhin eine von *Aspergillus oryzae* stammende Endopeptidase umfasst.

5.  Verfahren zur Gewinnung eines Fischproteinhydrolysats, wie in den Ansprüchen 1 bis 4 definiert, **dadurch gekennzeichnet, dass** es umfasst:

    - Zerkleinerung mindestens einer Proteinquelle, ausgewählt aus der Gruppe bestehend aus den Fischarten *Micromesistius poutassou, Clupea harengus, Scomber scombrus, Sardina pilchardus, Gadus morhua, Pollachius virens, Melanogrammus aeglefinus, Coryphaenoides rupestris, Trisopterus esmarki, Trachurus* spp., Fischen, die zur Ordnung der Siluriformes gehören, in Anwesenheit von Wasser, um die Pulpe des oder der Fischs/Fische zu gewinnen,
    - enzymatische Hydrolyse der Proteinquelle bei einer Temperatur zwischen 40 und 65°C, bei einem pH-Wert zwischen 6 und 9, für 1 bis 5 Stunden, nach Zugabe eines Enzymgemischs umfassend von *Bacillus amyloliquefaciens* und *Bacillus licheniformis* stammende Endopeptidasen, in einem Verhältnis Enzym/Proteinquelle zwischen 0,01 und 2%, um ein Reaktionsgemisch zu erhalten,
    - Anhalten der enzymatischen Hydrolyse durch Inaktivierung der Enzyme nach Temperaturerhöhung des Reaktionsgemischs auf nicht weniger als 70°C für 8 bis 20 Minuten,
    - Abtrennen des erhaltenen Proteinhydrolysats vom Rest der Reaktionsmischung.

6.  Verfahren gemäß Anspruch 5, **dadurch gekennzeichnet, dass** das Verhältnis Enzym/Proteinquelle 0,5% ist, die Hydrolysetemperatur 60°C ist und der pH-Wert 7,5 ist.

7.  Verfahren gemäß einem der Ansprüche 5 bis 6, **dadurch gekennzeichnet, dass** das Anhalten der enzymatischen Hydrolyse durchgeführt wird, wenn der Hydrolysegrad 8,9 erreicht.

8.  Verfahren gemäß einem der Ansprüche 5 bis 7, **dadurch gekennzeichnet, dass** das Enzymgemisch weiterhin eine von *Aspergillus oryzae* stammende Endopeptidase umfasst.

9.  Zusammensetzung, **dadurch gekennzeichnet, dass** sie ein Fischproteinhydrolysat, wie in den Ansprüchen 1 bis 4 definiert, umfasst.

10. Zusammensetzung gemäß Anspruch 9, **dadurch gekennzeichnet, dass** sie in Form eines Lebensmittels, eines Nahrungsergänzungsmittels oder einer nutrazeutischen Zusammensetzung vorliegt.

11. Pharmazeutische Zusammensetzung, **dadurch gekennzeichnet, dass** sie ein Fischproteinhydrolysat, wie in den Ansprüchen 1 bis 4 definiert, umfasst.

12. Verwendung eines Fischproteinhydrolysats, wie in den Ansprüchen 1 bis 4 definiert, zur Herstellung eines Medikaments zur Behandlung von Obesitas oder Typ-2-Diabetes.

13. Fischproteinhydrolysat, wie in den Ansprüchen 1 bis 4 definiert, zur Verwendung bei der Behandlung von Obesitas oder Typ-2-Diabetes.

14. Nicht-pharmazeutische Verwendung eines Fischproteinhydrolysats, wie in den Ansprüchen 1 bis 4 definiert, zur Steigerung des Sättigungsgefühls.

**Claims**

1.  Fish protein hydrolysate **characterised in that** it is obtained by enzymatic hydrolysis of at least one protein source chosen from the group composed of the fish species *Micromesistius poutassou, Clupea harengus, Scomber scombrus, Sardina pilchardus, Gadus morhua, Pollachius virens, Melanogrammus aeglefinus, Coryphaenoides rupestris, Trisopterus esmarki, Tracharus spp,* and fish species belonging to the order Siluriformes, the said enzymatic hydrolysis being carried out by means of a mixture of enzymes comprising endopeptidases derived from *Bacillus amyloliquefaciens* and *Bacillus licheniformis* and **in that** it has:

- the following molecular profile distribution: from 23% to 31% molecules with a molecular weight of less than 300 Da, from 31% to 34% molecules the molecular weight of which is between 300 and 1000 Da, from 28% to 34% molecules the molecular weight of which is between 1000 and 3000 Da, from 6% to 8% molecules the molecular weight of which is between 3000 and 5000 Da and 2% to 4% molecules the molecular weight of which is between 5000 and 10000 Da,
- a lipid content of less than 1% as a percentage of the raw product,
- a glucid content of less than 0.1% as a percentage of raw product,
- a protein content of more than 80% as a percentage of the raw product,
- a mineral matter content of between 10% and 20% as a percentage of raw product,

and **in that** it contains molecules immunologically similar to cholecystokinins (CCKs).

2. Fish protein hydrolysate according to claim 1, **characterised in that** it has the following amino acid composition: Glutamic acid 17.4%, Aspartic acid 11.4%, Lysine 10.2%, Leucine 8.4%, Arginine 6.1%, Alanine 6.8%, Valine 4.7%, Isoleucine 4.2%, Glycine 5%, Threonine 4.5%, Serine 4.4%, Tyrosine 3.2%, Phenylalanine 3.9%, Methionine 2.5%, Proline 3.6%, Histidine 1.9%, Cystine 1%, Tryptophan 0.8%, as a percentage by weight with respect to the total weight of amino acids.

3. Fish protein hydrolysate according to one of claims 1 to 2, **characterised in that** the said source of fish proteins comprises the pulp obtained from the fillet of the said fish or fishes.

4. Fish protein hydrolysate according to one of claims 1 to 3, **characterised in that** the said mixture of enzymes also comprises an endopeptidase derived from *Aspergillus oryzae.*

5. Method of obtaining a fish protein hydrolysate as defined in claims 1 to 4, **characterised in that** it comprises:

- the grinding of at least one protein source chosen from the group composed of the fish species *Micromesistius poutassou, Clupea harengus, Scomber scombrus, Sardina pilchardus, Gadus morhua, Pollachius virens, Melanogrammus aeglefinus, Coryphaenoides rupestris, Trisopterus esmarki, Tracharus spp,* and fish species belonging to the order Siluriformes, in the presence of water, so as to recover the pulp from the said fish or fishes,
- the enzymatic hydrolysis of the said protein source at a temperature of between 40° and 63°C, at a pH situated between 6 and 9, for 1 to 5 hours, after the addition of a mixture of enzymes comprising endopeptidases derived from *Bacillus amyloliquefaciens* and *Bacillus licheniformis,* in a ratio of enzyme to protein source of between 0.01 and 2%, so as to obtain a reaction mixture,
- stoppage of the said enzymatic hydrolysis by inactivation of the said enzymes after raising the temperature of the said reaction mixture to a level not below 70°C, for 8 to 20 minutes,
- the separation of the protein hydrolysate obtained from the rest of the reaction mixture.

6. Method according to claim 5, **characterised in that** the said enzyme/protein source ratio is 0.5%, the said hydrolysis temperature is 60°C and the said pH is 7.5.

7. Method according to one of claims 5 to 6, **characterised in that** the said stoppage of the said enzymatic hydrolysis is done when the degree of hydrolysis reaches 8.9.

8. Method according to one of claims 5 to 7, **characterised in that** the said mixture of enzymes also contains an endopeptidase derived from *Aspergillus oryzae.*

9. Composition **characterised in that** it comprises a fish protein hydrolysate as defined in claims 1 to 4.

10. Composition according to claim 9, **characterised in that** it is in the form of a food product, a food supplement or a neutraceutical composition.

**11.** Pharmaceutical composition **characterised in that** it comprises a fish protein hydrolysate as defined in claims 1 to 4.

**12.** Use of a fish protein hydrolysate as defined in claims 1 to 4 for manufacturing a medication intended for the treatment of obesity or type II diabetes.

**13.** Fish protein hydrolysate as defined in claims 1 to 4 for use thereof in the treatment of obesity or type II diabetes.

**14.** Non pharmaceutical use of a fish protein hydrolysate as defined in claims 1 to 4 for increasing the control of satiety.

**Fig. 1**

**Fig. 2**

**Fig. 3**

**Fig. 4**

PL 3/6

Fig. 5

Fig. 6

Fig. 7

Fig. 8

**Fig. 9**

**Fig. 10**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **RAVALLEC-PLÉ R. et al.** *Comparative Biochemistry and Physiology,* 2003, vol. 134 (4), 669-679
- **STRADER AD ; WOODS SC.** *Gastrointestinal hormones and food intake. Gastroenterology,* 2005, vol. 128, 175-191
- **MORAN TH ; KINZIG KP.** Gastrointestinal satiety signals II. Cholecystokinin. *Am. J. Physiol. Gastrointest. Liver Physiol.,* 2004, vol. 286, G183-188
- **MORAN TH.** Cholecystokinin and satiety: current perspectives. *Nutrition,* 2000, vol. 16, 858-865
- **CHAUDHRI O ; SMALL C ; BLOOM S.** Gastrointestinal hormones regulating appetite. *Philos Trans R Soc Lond B Biol Sci,* 2006, vol. 361, 1187-209
- **CUBER JC ; BERNARD C ; LEVENEZ F ; CHAYVIALLE JA.** Lipids, proteins and carbohydrates stimulate la sécrétion of intestinal cholecystokinin in the pig. *Reprod Nutr Dev,* 1990, vol. 30, 267-75
- **BAILE CA ; MCLAUGHLIN CL ; DELLA-FERA MA.** Role of cholecystokinin and opioid peptides in control of food intake. *Physiol Rev,* 1986, vol. 66, 172-234
- **LIDDLE RA ; GREEN GM ; CONRAD CK ; WILLIAMS JA.** Proteins but not amino acids, carbohydrates, or fats stimulate cholecystokinin secretion in the rat. *Am J Physiol,* 1986, vol. 251, G243-8
- **DOUGLAS BR ; WOUTERSEN RA ; JANSEN JB et al.** The influence of different nutrients on plasma cholecystokinin levels in the rat. *Experientia,* 1988, vol. 44, 21-3
- **MIAZZA B ; PALMA R ; LACHANCE JR et al.** Jejunal secretory effect of intraduodenal food in humans. A comparison of mixed nutrients, proteins, lipids, and carbohydrates. *Gastroenterology,* 1985, vol. 88, 1215-22
- **MANGEL AW et al.** Phenylalanine-stimulated sécrétion of cholecystokinin is calcium dependent. *Am J Physiol,* 1995, vol. 268 (1), G90-4
- **BRUBAKER PL ; IZZO A ; ROCCA AS.** Synthesis and secretion of intestinal proglucagon-derived peptides by the STC-1 enteroendocrine cell line. *Can J Diabetes.,* 2003, vol. 27, 141-148